# DEMANDE DE BREVET EUROPEEN

(11) **EP 2 460 561 A1**
(43) Date de publication de la demande: **06.06.2012**
(21) Numéro de dépôt: 12153664.3
(22) Date de dépôt: 21.12.2007
(51) Int. Cl.: A61Q 19/08, A61Q 7/00, A61Q 17/04, A61Q 19/00, A61K 8/36, A61K 8/38, A61K 31/327, A61P 17/00, A61K 31/185, A61K 8/04, A61K 8/06, A61K 8/37, A61K 8/67, A61K 31/192, A61K 31/00

(54) **Emulsion comprenant au moins un retinoide et du péroxyde de benzoyle**

(30) Priorité: 21.12.2006 FR 0655783
(62) Demande divisionnaire de: 07872016.6
(71) Demandeur: Galderma Research & Development, 06410 Biot (FR)
(72) Inventeur: Mallard, Claire, 06250 Mougins (FR); Louis, Fabienne, 06270 Villeneuve-Loubet (FR); Willcox, Nathalie, 06460 Saint Vallier De Thiey (FR)
(74) Mandataire: Boulard, Denis

(57) **Abrégé**

L'invention se rapporte à une composition sous forme d'émulsion comprenant, dans un milieu physiologiquement acceptable, au moins un rétinoïde et du péroxyde de benzoyle dispersé, à son procédé de préparation et à son utilisation en cosmétique et en dermatologie.

## Description

L'invention se rapporte à une composition sous forme d'émulsion comprenant, dans un milieu physiologiquement acceptable, au moins un rétinoïde et du péroxyde de benzoyle dispersé.

L'utilisation de plusieurs classes de principes actifs est un outil thérapeutique auquel il est fréquemment fait recours, notamment pour le traitement de désordres dermatologiques. En effet, différents antifongiques comme les dérivés allylamines, les triazoles, les antibactériens ou antimicrobiens comme par exemple les antibiotiques, les quinolones et les imidazoles, sont classiquement associés dans le traitement de maladies dermatologiques. Il est également connu d'utiliser les péroxydes, les vitamines D et les rétinoïdes pour le traitement topique de diverses pathologies liées à la peau ou les muqueuses, en particulier l'acné.

La combinaison de plusieurs traitements locaux (antibiotiques, rétinoïdes, péroxydes, zinc) est également utilisée en dermatologie pour permettre d'augmenter l'efficacité des principes actifs et de diminuer leur toxicité (Cunliffe W.J., J. Dermatol. Treat., 2000, 11 (suppl2), S13-S14).

L'application multiple de différents produits dermatologiques peut être assez lourde et astreignante pour le patient.

On comprend donc l'intérêt de chercher à obtenir un nouveau traitement efficace sur les affections dermatologiques dans une composition stable offrant une bonne cosméticité, permettant une application unique et une utilisation agréable pour le patient.

Parmi cette panoplie de thérapeutiques proposée à l'homme du métier, rien ne l'encourageait à associer, dans la même composition, le péroxyde de benzoyle et un rétinoïde.

Toutefois, la formulation d'une telle composition pose plusieurs problèmes.

Tout d'abord, l'efficacité du péroxyde de benzoyle est liée à sa décomposition lorsqu'il est mis en contact avec la peau. En effet, ce sont les propriétés oxydantes des radicaux libres produits lors de cette décomposition qui conduisent à l'effet désiré. Aussi, afin de maintenir au péroxyde de benzoyle une efficacité optimale, il est important de prévenir sa décomposition avant utilisation, c'est à dire durant le stockage.

Or le péroxyde de benzoyle est un composé chimique instable qui rend difficile sa formulation dans des produits finis.

La solubilité et la stabilité du péroxyde de benzoyle ont été étudiées par Chellquist *et al.* dans l'éthanol, le propylène glycol et différents mélanges de polyéthylène glycol 400 (PEG 400) et d'eau (Chellquist E.M. et Gorman W.G., Pharm. Res., 1992, Vol 9: 1341-1346). Le péroxyde de benzoyle est particulièrement soluble dans le PEG400 et l'éthanol comme le montre le tableau suivant :

| **Solvant** | Solubilité du péroxyde de benzoyle (mg/g) |
|---|---|
| PEG 400 | 39.6 |
| Ethanol | 17.9 |
| Propylène Glycol | 2.95 |
| Propylène Glycol / Eau (75 :25) | 0.36 |
| Glycérine | 0.15 |
| Eau | 0.000155 |

Ce document précise par ailleurs que la stabilité du péroxyde de benzoyle est fortement influencée par la composition chimique de la formulation et par la température de stockage. Le péroxyde de benzoyle est extrêmement réactif et se dégrade en solution à basse température en raison de l'instabilité de sa liaison péroxyde.

Les auteurs constatent ainsi que le péroxyde de benzoyle en solution se dégrade plus ou moins rapidement dans tous les solvants étudiés en fonction du type de solvant et de sa concentration.

Les temps de dégradation du péroxyde de benzoyle dans le PEG 400 (0.5 mg/g), dans l'éthanol et dans le propylène glycol sont respectivement de 1,4 , 29 et 53 jours à 40°C. Une telle dégradation ne permet pas la préparation d'un produit destiné à la vente.

Il est connu par ailleurs que le péroxyde de benzoyle est plus stable dans l'eau et le propylène glycol lorsqu'il est en suspension (ie sous forme dispersée), puisqu'il n'est pas dégradé après 90 jours de conservation dans ces solvants.

Ainsi, pour limiter le problème d'instabilité rapide du péroxyde de benzoyle en solution, il s'est avéré avantageux de formuler le péroxyde de benzoyle sous forme dispersée. Cependant, ce type de formulation n'est pas totalement satisfaisant dans la mesure où on constate toujours une dégradation du péroxyde de benzoyle dans le produit fini.

Une autre difficulté à surmonter pour la préparation d'une composition comprenant à la fois du péroxyde de benzoyle et un rétinoïde est que la plupart des rétinoïdes sont particulièrement sensible à l'oxydation naturelle, à la lumière visible et aux ultra-violets, et le péroxyde de benzoyle étant un oxydant fort, la compatibilité chimique de ces composés dans une même formulation pose de nombreux problèmes de stabilité du point de vue physique et chimique.

Une étude de stabilité de deux rétinoïdes a été réalisée en combinant deux produits commercialisés, l'un contenant un rétinoïde (trétinoïne ou adapalène) et le second à base de péroxyde de benzoyle *(*B. Martin et al., Br.J.Dermatol. (1998) 139, (suppl.52), 8-11*).* La présence de la formulation à base de péroxyde de benzoyle provoque une dégradation très rapide des rétinoïdes sensibles à l'oxydation : on mesure que 50% de la trétinoïne se dégrade en 2 heures, et 95% en 24 heures. Dans la composition dans laquelle le rétinoïde est l'adapalène, aucune dégradation de l'adapalène n'a été mesurée pendant 24 heures. Cette étude confirme que le péroxyde de benzoyle se dégrade et dégrade les rétinoïdes sensibles à l'oxydation au cours du temps en relarguant progressivement de l'acide benzoïque dans des produits finis.

Or il est clair que la dégradation du péroxyde de benzoyle et des rétinoïdes n'est pas souhaitable dans la mesure où elle nuit à l'efficacité de la composition les contenant.

Rien n'incitait à associer ces deux agents actifs afin d'obtenir une composition stable de type émulsion sachant qu'il était usuellement connu que la présence du péroxyde de benzoyle déstabilisait chimiquement et physiquement ce type de composition.

La formulation en émulsion « légère » du péroxyde de benzoyle et d'un rétinoïde est avantageuse pour les traitements topiques, tels que celui de l'acné, car tout en apportant l'émollience, elle évite notamment de laisser subsister un toucher trop gras sur la peau. Par émulsion légère on entend une émulsion contenant une faible proportion de phase grasse, la phase aqueuse restant majoritaire.

On entend par émulsion, un système liquide comportant deux fluides insolubles ou peu solubles l'un dans l'autre, et dans lequel un des fluides se disperse dans l'autre en particules microscopiques. De préférence, les émulsions utilisées comprennent au moins un émulsionnant, une phase hydrophile, de préférence aqueuse, polaire et une phase grasse non polaire. De préférence, elles se présentent sous forme d'émulsions « huile dans l'eau » (H/E), ou « eau dans l'huile » (E/H).

Une crème est une formule qui contient de l'eau et de l'huile, et est stabilisée avec un émulsionnant. Les crèmes lipophiles sont des émulsions dites « eau dans l'huile » (E/H), les crèmes hydrophiles sont appelées émulsions « huile dans l'eau » (H/E).Les crèmes E/H ont généralement des bases absorbantes (vaseline, cérésine, lanoline...). Les crèmes H/E ont pour base des mono-, di- et triglycérides d'acides gras ou d'alcools gras avec du savon, des sulfates alkyles ou des éthers de polycyclols alkyles utilisés comme émulsionnants.

Les crèmes peuvent recréer un film hydrolipidique perturbé ou réhydrater la couche cornée grâce à leur pouvoir occlusif. Elles peuvent également servir de nettoyants protecteurs ou de véhicules pour transporter des substances thérapeutiques.

Or, une autre difficulté à surmonter pour la préparation d'une telle composition comprenant notamment des actifs dispersés tels que l'Adapalène et le péroxyde de benzoyle est la sédimentation des actifs. Car si le toucher final d'une telle formulation est évidement lié au sens de l'émulsion et à sa composition, une émulsion légère sera plutôt orientée H/E et les corps gras tels que la vaseline, les cires et huiles minérales seront limités, la viscosité jouant un rôle important. Ainsi une émulsion légère sera préférentiellement fluide voire pulvérisable. Dans le cas d'une émulsion plus visqueuse, les épaississants de la phase grasse tels que les alcools et esters gras solides seront fortement diminués au profit de gélifiants dans la phase aqueuse. Or la plupart des gélifiants de la phase aqueuse sont déstabilisés par l'acide benzoïque libéré lors de la dégradation du péroxyde de benzoyle.

En effet, les agents épaississants les plus couramment utilisés pour la formulation de gels avec du péroxyde de benzoyle sont les polymères d'acide acrylique (Carbomer) et les celluloses seules ou associées à des silicates.

Or, l'utilisation de carbomères dans des compositions de type gel aqueux ne donne pas de bons résultats en terme de stabilité chimique du péroxyde de benzoyle et en terme de stabilité rhéologique. Comme décrit par Bollinger (Bollinger, Journal of Pharmaceutical Science, 1977, vol 5), il a été observé une perte de 5 à 20% de péroxyde de benzoyle au bout de 2 mois à 40°C selon le neutralisant du carbomère utilisé. De plus, la libération d'acide benzoïque provoque la dépolymérisation des carbomères, donnant une chute de viscosité pouvant entraîner un déphasage.

Cette instabilité des gels de péroxyde de benzoyle nuit à leur efficacité et à leur cosméticité et il est fortement probable qu'on la retrouve dans les émulsions gélifiées. Par ailleurs, un produit fini, en particulier lorsqu'il s'agit de compositions pharmaceutiques ou cosmétiques, doit conserver tout au long de sa durée de vie des critères physico-chimiques précis permettant de garantir sa qualité pharmaceutique ou cosmétique, respectivement. Parmi ces critères, il est nécessaire que les propriétés rhéologiques soient conservées. Elles définissent le comportement et la texture de la composition lors de l'application, mais aussi les propriétés de libération du principe actif [Rapport commission SFSTP 1998] et l'homogénéité du produit lorsque les principes actifs y sont présents à l'état dispersé.

Il existe donc le besoin de disposer d'une crème ou d'une émulsion fluide type lotion physiquement et chimiquement stable comprenant du péroxyde de benzoyle et un rétinoïde.

Or, la Demanderesse a réalisé une composition satisfaisant ce besoin sous forme d'une émulsion qui comprend du péroxyde de benzoyle dispersé, notamment sous forme libre ou encapsulée, au moins un rétinoïde, au moins un émulsionnant, et, lorsqu'elle est gélifiée, un gélifiant pH-indépendant et ayant une bonne stabilité physique, c'est-à-dire ne présentant pas de chute de viscosité dans le temps et à des températures comprises entre 4°C et 40°C, et maintenant une bonne stabilité chimique des deux actifs (péroxyde de benzoyle et rétinoïde), c'est-à-dire qu'on ne constate pas de dégradation des actifs dans le temps et à des températures comprises entre 4°C et 40°C.

La Demanderesse a également découvert de manière surprenante que l'on pouvait obtenir une parfaite dispersion des principes actifs en suivant un procédé de préparation particulier. Ce procédé de préparation permet d'obtenir une granulométrie optimale et une dispersion homogène des deux actifs dans la composition, tout en garantissant la stabilité physique du produit.

L'invention se rapporte donc à une composition sous forme d'émulsion comprenant, dans un milieu physiologiquement acceptable, au moins un rétinoïde et du péroxyde de benzoyle.

Par milieu physiologiquement acceptable, on entend un milieu compatible avec une application topique sur la peau, les phanères et/ou les muqueuses.

Les compositions de la présente invention peuvent se présenter sous toutes les formes galéniques normalement utilisées pour une application topique et notamment sous forme d'émulsions de consistance liquide (notamment compatible avec une présentation de type lingettes imprégnées) ou semi-liquide du type lait, obtenues par dispersion d'une phase grasse dans une phase aqueuse (H/E) ou d'émulsions de consistance molle, semi-liquide ou semi-solide du type crème.

L'homme du métier veillera à choisir les excipients constituant les compositions selon l'invention en fonction de la forme galénique souhaitée et de manière à ce que les propriétés avantageuses de la composition selon l'invention soient respectées.

La demanderesse a en particulier réalisé une émulsion comprenant :
- un rétinoïde et le péroxyde de benzoyle, notamment sous forme libre ou encapsulée ;
- au moins une phase hydrophile;
- au moins une phase grasse ;
- au moins un émulsionnant.

Plus particulièrement la phase hydrophile de la composition selon l'invention telle que défini plus haut est une phase aqueuse.

Les compositions selon l'invention sont plus particulièrement stables physiquement et chimiquement.

La composition selon l'invention peut notamment comprendre, outre au moins un rétinoïde, du péroxyde de benzoyle et au moins un émulsionnant, un ou plusieurs des ingrédients suivants :
a) un ou plusieurs agents gélifiants et/ou agents de suspension et/ou gélifiants pH indépendants,
b) un ou plusieurs agents chélatants,
c) un ou plusieurs agents mouillants,
d) un ou plusieurs excipients lipophiles composant la phase grasse,
e) une phase aqueuse,
f) un ou plusieurs additifs.

La composition selon l'invention est de préférence sous la forme d'une émulsion huile dans eau (H/E).

Pour obtenir cette stabilisation indispensable, on introduit un émulsionnant qui réduit la tension de surface entre les deux phases. Parce qu'elles correspondent aux besoins physiologiques de la peau, et permettent de faire pénétrer uniformément les substances solubles dans l'eau comme celles qui sont solubles dans l'huile, les émulsions ont un rôle important dans les produits dermatologiques et cosmétiques.

Les concentrations préférées en émulsionnants sont comprises entre 0,001 et 20 % en poids par rapport au poids total de la composition. De préférence encore la concentration est comprise entre 1 et 15% et de manière préférée entre 3 et 11 % en poids par rapport au poids total de la composition.

Les émulsionnants sont des composés amphiphiles qui possèdent une partie hydrophobe ayant une affinité pour l'huile et une partie hydrophile ayant une affinité pour l'eau créant ainsi un lien entre les deux phases. Les émulsionnants ioniques ou non ioniques stabilisent donc les émulsions H/E en s'adsorbant à l'interface et en formant des couches lamellaires de cristaux liquides.

Le pouvoir émulsifiant des émulsionnants non-ioniques est étroitement lié à la polarité de la molécule. Cette polarité est définie par le HLB (Balance Hydrophile/Lipophile).

Une HLB élevée indique que la fraction hydrophile est prédominante, et, à l'inverse, une faible HLB indique que la partie lipophile est prédominante. Par exemple, des valeurs de HLB supérieures à environ 10 correspondent à des tensioactifs hydrophiles.

Les émulsionnants peuvent être classés, selon leur structure, sous les termes génériques "ioniques" (anioniques, cationiques, amphotères) ou "non ioniques". Les émulsionnants non ioniques sont des émulsionnants qui ne se dissocient pas en ions dans l'eau et sont donc insensibles aux variations de pH.

Les émulsionnants non ioniques sont particulièrement bien adaptés pour la préparation d'émulsions de type huile-dans-eau, objet de la présente invention. Ainsi, le système émulsionnant, composant de l'émulsion de l'invention, comprend au moins un émulsionnant non ionique, à fraction prédominante hydrophile, c'est-à-dire présentant une HLB élevée, supérieure à environ 10.

On peut citer, à titre d'exemple non limitatif des émulsionnants non ioniques présentant une HLB élevée, les esters de sorbitan tels que le POE(20) sorbitan monooléate, vendu sous le nom de Tween 80 ® (HLB=15) ; le POE(20) sorbitan monostéarate vendu sous le nom de Tween 60 ® (HLB=14.9) ; les éthers d'alcools gras tels que le POE (21) stéaryl ether (HLB= 15.5) vendu sous le nom de Brij 721® par la société Uniqema, ou le ceteareth 20 vendu sous le nom de Eumulgin B2® (HLB de 15,5) par la société Cognis, les esters de polyoxyethylene glycol tel que le glyceryl stearate and PEG 100 stéarate vendu sous le nom de Arlacel 165 FL ® (HLB=11) par la société Uniqema , le PEG 6 Stéarate et PEG 32 stéarate vendu sous le nom de TEFOSE 1500 ® (HLB= 10) par la société Gateffossé, les sucroesters de haut HLB tel que le PEG 20 methyl glucose sesquistéarate vendu sous les noms de glucamate SSE20 ® (HLB=15) par la société Amerchol et le sucrose laurate vendu sous le nom de Surfhope C-1216® (HLB=16) et le sucrose stéarate vendu sous le nom de Surfhope C-1811® (HLB=11) par la société Gattefossé. De préférence, lesdits émulsionnants non ioniques de haute HLB, présentent une HLB comprise entre 10 et 18.

On citera, comme exemples non limitatifs, les émulsionnants non ioniques de basse HLB (lipophiles), les esters de sorbitan, tels que le monostéarate de sorbitan (HLB = 4.7) vendu sous le nom de Span 60 par la société Uniqema, les esters de glycérol tel que le monostéarate de glycerol vendu sous le nom de Cutina GMSVPH (HLB=3.8) par la sociéta Cognis, les esters de polyethylène glycol tel que le PEG-6 isostéarate vendu sous le nom de Olepal isostéarique® (HLB=8) par la société Gattefossé, les sucroesters de bas HLB tel que le methyl glucose sesquistéarate vendu sous le nom de Glucate SS® (HLB= 6). par la société Amerchol et le sucrose dilaurate vendu sous le nom de Surfhope C-1205 (HLB=5) et le sucrose tristéarate vendu sous le nom de Surfhope C-1803® (HLB=3) par la société Gattefossé.

De préférence, lesdits émulsionnants non ioniques présentant de faible HLB, présentent une HLB inférieure à 10.

Les émulsionnants non ioniques peuvent être utilisés seuls ou en mélange de deux ou plusieurs d'entre eux pour former le système émulsionnant composant l'émulsion de l'invention.

De préférence, on utilisera, en tant que système émulsionnant un ou plusieurs couples " émulsionnant non ionique de haute HLB" / " émulsionnant non ionique de faible HLB", il pourra en particulier s'agir d'un système émulsionnant non ionique comprenant au moins un émulsionnant non ionique présentant une HLB supérieure à environ 10 et au moins un tensioactif non ionique présentant une HLB inférieure à environ 10.

Le ratio de chacun des deux émulsionnants formant le couple précité est déterminé le plus souvent par le calcul de la HLB requise de la phase grasse utilisée.

A titre d'émulsionnants préférés on peut citer des émulsionnants hydrophiles de type, Glyceryl Stéarate & PEG-100 Stéarate vendu sous le nom Arlacel 165FL® par la société Uniqema; le PEG 6 stéarate et PEG 32 stéarate vendu sous le nom de TEFOSE 1500® par Gattefossé, le PEG 20 methyl glucose sesquistéarate vendu sous le nom de Glucamate SSE 20® par Amerchol, le Polyoxyethylene (21) Stéaryl Ether vendu sous le nom Brij721® par Uniqema,et le Cétéareth 20 vendu sous le nom d'Eumulgin B2PH® par Cognis ; des émulsionnants lipophiles de type methyl glucose sesquistéarate tels que le Glucate SS® vendu par Amerchol.

La composition selon l'invention contient au moins un rétinoïde. Par rétinoïde, on entend tout composé se liant aux récepteurs RAR et/ou RXR.

A titre d'exemple on peut citer comme rétinoïde, l'acide rétinoique, la trétinoine, le tazarotène ainsi que ceux décrits dans les brevets ou demandes de brevet suivants :
US 4,666,941, US 4,581,380, EP 0 210 929, EP 0 232 199, EP 0 260 162, EP 0 292 348,
EP 0 325 540, EP 0 359 621, EP 0 409 728, EP 0 409 740, EP 0 552 282, EP 0 584 191,
EP 0 514 264, EP 0 514 269, EP 0 661 260, EP 0 661 258, EP 0 658 553, EP 0 679 628,
EP 0 679 631, EP 0 679 630, EP 0 708 100, EP 0 709 382, EP 0 722 928, EP 0 728 739,
EP 0 732 328, EP 0 740 937, EP 0 776 885, EO 0 776 881, EP 0 823 903, EP 0 832 057,
EP 0 832 081, EP 0 816 352, EP 0 826 657, EP 0 874 626, EP 0 934 295, EP 0 915 823,
EP 0 882 033, EP 0 850 909, EP 0 879 814, EP 0 952 974, EP 0 905 118, EP 0 947 496, WO98/56783, WO99/10322, WO99/50239, WO99/65872.

Par leur capacité à lier les récepteurs RAR et/ou RXR, les composés issus de la famille des rétinoïdes benzonaphtaléniques tels que décrits dans la demande de brevet EP 0 199 636 sont également inclus dans l'invention.

Préférentiellement, on choisira les dérivés de l'acide naphtoïque et notamment :
L'acide 6-(3-méthylphényl)-2-naphtoïque et son ester méthylique,
L'acide 6-(4-tertiobutylphényl)-2-naphtoïque et son ester méthylique,
L'acide 6-(3-tertiobutylphényl)-2-naphtoïque et son ester méthylique,
L'acide 6-(3,4-diméthoxyphényl)-2-naphtoïque et son ester méthylique,
L'acide 6-(p-(1-adamantylthio)phényl)-2-naphtoïque et son ester méthylique,
L'acide 6-(3-(1-adamantyl)-4-méthoxyphényl)-2-naphtoïque (adapalène) et son ester méthylique,
L'ester méthylique de l'acide 6-[3-(1-adamantyl)-4-tert-butyldiméthylsilyloxyphényl)-2-naphtoïque,
L'ester méthylique de l'acide 6-[3-(1-adamantyl)-4-hydroxyphényl)-2-naphtoïque,
L'acide 6-[3-(1-adamantyl)-4-hydroxyphényl)-2-naphtoïque,
L'ester méthylique de l'acide 6-[3-(1-adamantyl)-4-décyloxyphényl)-2-naphtoïque,
L'acide 6-[3-(1-adamantyl)-4-décyloxyphényl)-2-naphtoïque,
L'ester méthylique de l'acide 6-[3-(1-adamantyl)-4-hexyloxyphényl)-2-naphtoïque,
L'acide 6-[3-(1-adamantyl)-4-hexyloxyphényl)-2-naphtoïque,
L'ester méthylique de l'acide 6-[3-(1-adamantyl)-4-méthoxyphényl)-4-acétoxy-1-méthyl-2-naphtoïque,
L'acide 6-[3-(1-adamantyl)-4-méthoxyphényl)-4-hydroxy-1-méthyl-2-naphtoïque,
L'ester méthylique de l'acide 6-[3-(1-adamantyl)-4-méthoxyphényl)-4-hydroxy-1-méthyl-2-naphtoïque,
L'ester méthylique de l'acide 6-[3-(1-adamantyl)-4-méthoxyphényl)-1-méthyl-2-naphtoïque,
L'acide 6-[3-(1-adamantyl)-4-méthoxyphényl)-1-méthyl-2-naphtoïque,
Le 6-[3-(1-adamantyl)-4-méthoxyphényl)-2-naphtalène méthanol,
L'éthylamide de l'acide 6-[3-(1-adamantyl)-4-méthoxyphényl)-2-naphtoïque,
Le morpholide de l'acide 6-[3-(1-adamantyl)-4-méthoxyphényl)-2-naphtoïque,
L'ester méthylique de l'acide 6-[3-tert-butyl-4-méthoxyphényl)-2-naphtoïque,
L'acide 6-[3-tert-butyl-4-méthoxyphényl)-2-naphtoïque,
L'ester méthylique de l'acide 6-[3-(1,1-diméthyldécyl)-4-méthoxyphényl)-2-naphtoïque,
L'acide 6-[3-(1,1-diméthyldécyl)-4-méthoxyphényl)-2-naphtoïque.

En particulier, on préférera l'adapalène ainsi que ses sels.

On entend par sels de l'adapalène, les sels formés avec une base pharmaceutiquement acceptable, notamment des bases minérales telles que la soude, la potasse et l'ammoniaque ou des bases organiques telles que la lysine, l'arginine, la N-méthyl-glucamine.

On entend également par sels de l'adapalène les sels formés avec des amines grasses telles que la dioctylamine et la stéarylamine.

Bien entendu, la quantité des deux agents actifs, péroxyde de benzoyle et rétinoïde, dans la composition selon l'invention dépendra de l'association choisie et donc particulièrement du rétinoïde considéré et de la qualité du traitement désiré.

Les concentrations en rétinoïde préférées sont comprises entre 0,0001 et 20 % en poids par rapport au poids total de la composition.

De manière préférée, dans le cas de l'adapalène, la composition selon l'invention comprend entre 0.001 et 5% et avantageusement entre 0.01 et 1 % en poids d'adapalène par rapport au poids total de la composition, préférentiellement entre 0.01 et 0.5%, de préférence, entre 0.1 et 0.4 % en poids d'adapalène, encore plus préférentiellement 0.3 % en poids d'adapalène.

Le péroxyde de benzoyle pourra aussi bien être utilisé sous la forme libre ou bien sous une forme encapsulée par exemple sous forme adsorbée sur, ou absorbée dans tout support poreux. Il peut s'agir par exemple de péroxyde de benzoyle encapsulé dans un système polymérique constitué de microsphères poreuses, comme par exemple des microéponges vendues sous le nom de Microsponges P009A Benzoyle péroxyde ® par la société Cardinal Health.

Pour donner un ordre de grandeur, la composition selon l'invention comprend avantageusement entre 0,0001 et 20 % en poids de péroxyde de benzoyle et entre 0,0001 et 20 % en poids de rétinoïde par rapport au poids total de la composition, et de préférence, respectivement, entre 0,025 et 10 % en poids de péroxyde de benzoyle et entre 0,001 et 10 % en poids de rétinoïde par rapport au poids total de la composition.

Par exemple, dans les compositions pour le traitement de l'acné, le péroxyde de benzoyle est utilisé, de préférence, à des concentrations allant de 2 à 10 % en poids et plus particulièrement de 2,5 à 5 % en poids par rapport au poids total de la composition. Le rétinoïde est quant à lui utilisé dans ce type de composition à des concentrations allant généralement de 0,01 à 1 % en poids par rapport au poids total de la composition.

De façon avantageuse, la granulométrie du rétinoïde et du péroxyde de benzoyle est telle qu'au moins 80% en nombre des particules, et de préférence au moins 90% en nombre des particules, ont un diamètre inférieur à 25 µm et au moins 99% en nombre des particules ont un diamètre inférieur à 100 µm.

Les compositions de l'invention peuvent comprendre un ou plusieurs agents mouillants. Ces ingrédients peuvent être présents dans des concentrations allant de 0,001 à 20 %, préférentielles de 0,1% à 10% et plus préférentiellement allant de 2 à 7 % en poids par rapport au poids total de la composition. Ils doivent être non solubilisant des actifs au pourcentage utilisé, ne pas provoquer de réactions exothermiques néfastes pour le péroxyde de benzoyle, aider à la bonne dispersion des actifs et avoir des propriétés anti-mousses.

Le pouvoir mouillant est la tendance d'un liquide à s'étaler sur une surface solide. En diminuant la tension superficielle, les agents mouillants permettent un plus grand étalement du liquide et aident ainsi à la dispersion des particules solides de rétinoïdes et de peroxyde de benzoyle.

De préférence, il s'agit d'agents mouillants pouvant présenter une HLB (Hydrophilic Lipophilic Balance) de 7 à 18 ou bien des tensioactifs non ioniques de type copolymères polyoxyéthylénés et/ou polyoxypropylénés à titre d'exemple non limitatif, on citera les Poloxamers et plus particulièrement le Synperonic PE/L44 et/ou le Synperonic PE/L62 vendus par la société Uniqema, des glycols tels que le propylene glycol, le dipropylène glycol, le lauroglycol, le propylène glycol dipélargonate, l'éthoxydiglycol. De préférence, les agents mouillants sont sous forme liquide de manière à s'incorporer aisément dans la composition sans qu'il soit nécessaire de la chauffer.

Parmi les agents mouillants, on utilise préférentiellement, un agent mouillant pouvant présenter préférentiellement une HLB de 10 à 14, sans que cette liste soit limitative, des composés de la famille des Poloxamers et/ou des glycols et plus particulièrement le Synperonic PE/L44 et/ou le Synperonic PE/L62 et/ou des composés tels que le propylène glycol, le dipropylène glycol, le propylène glycol dipélargonate, le lauroglycol, l'éthoxydiglycol,

L'agent mouillant particulièrement préféré est le propylène glycol et le Synperonic PE/L44 (Polyethylene-polypropylene glycol; Polyoxyethylene-Polyoxypropylene Block Copolymer).

Selon l'invention, d'une manière avantageuse, l'émulsion comprenant le péroxyde de benzoyle et un rétinoïde comprend au moins de l'eau et peut également comprendre un agent propénétrant et/ou un agent mouillant.

La composition selon l'invention comprend également une phase grasse. Cette phase grasse peut comprendre par exemple, les huiles végétales, minérales, animales ou synthétiques, des huiles de silicones, et leurs mélanges.

Comme exemple d'huile minérale, on peut citer par exemple des huiles de paraffine de différentes viscosités telles que le Primol 352®, le Marcol 82®, Marcol 152® vendus par la société Esso.

Comme huile végétale, on peut citer l'huile d'amande douce, l'huile de palme, l'huile de soja, l'huile de sésame, l'huile de tournesol.

Comme huile animale, on peut citer la lanoline, le squalène, l'huile de poisson, l'huile de vison avec comme dérivé le squalane vendu sous le nom Cosbiol® par la société Laserson.

Comme huile synthétique, on peut citer un ester tel que le cétéaryl isononanoate comme le produit vendu sous le nom de Cetiol SN PH® par la société Cognis France, , le palmitate d'isopropyle comme le produit vendu sous le nom de Crodamol IPP® par la société Croda, le diisopropyl adipate vendu sous le nom de Crodamol DA par la société Croda, le caprylique/caprique triglycéride tel que Miglyol 812® vendu par la société Hüls / Univar.

Comme huile de silicone volatile ou non volatile, on peut citer des diméthicones comme les produits vendus sous le nom de Q7-9120 silicone fluid de viscosité 20 cst à 12500 cst ou le produit vendu sous le nom ST-Cyclomethicone-5 NF® par la société Dow corning.

On pourra également mettre des corps gras solides tel que des cires naturelles ou synthétiques, des acides gras tel que l'acide stéarique , des alcools gras tel que le Speziol C18 pharma vendu par la société Cognis, et des agents de texture de type tribehenate, tel que le Compritol 888 vendu par la société Gattefossé ou les huiles de ricin hydrogénées tel que le Cutina HR vendu par la société Cognis . Dans ce cas, l'homme du métier adaptera la température de chauffage de la préparation en fonction de la présence ou non de ces solides.

Pour la composition selon l'invention, les huiles synthétiques et les huiles de silicone et plus particulièrement le Miglyol 812® et la ST-Cyclomlethicone 5 NF® sont préférés.

La phase hydrophile de l'émulsion selon l'invention est de préférence aqueuse et donc peut comprendre de l'eau. Cette eau peut être notamment une eau florale telle que l'eau de bleuet, ou une eau thermale ou minérale naturelle, par exemple choisie parmi l'eau de Vittel, les eaux du bassin de Vichy, l'eau d'Uriage, l'eau de la Roche Posay, l'eau d'Avène ou l'eau d'Aix les Bains.

Ladite phase aqueuse peut être présente à une teneur comprise entre 10 et 90 % en poids par rapport au poids total de la composition, de préférence comprise entre 20 et 80 % en poids.

Parmi les agents chélatants, on peut citer à titre d'exemples non limitatifs l'acide éthylène diamine tétraacétique (EDTA), l'acide diéthylène triamine pentaacétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA) et l'acide éthylène diamine-di (5-carboxy-2-hydroxyphényl) acétique (EDDCHA).

Les concentrations d'agent chélatant peuvent varier de 0% à 1,5% préférentiellement 0,05% à 0.5% en poids par rapport au poids total de la composition.

A titre d'agent chélatant préféré, on peut citer l'acide éthylène diamine tétraacétique (EDTA)

A titre d'exemple non limitatif de gélifiants et/ou agents de suspension et/ou agents gélifiants pH indépendant, pouvant entrer dans les compositions selon l'invention, on peut citer, la microscrystalline cellulose et carboxymethyl cellulose de sodium vendu sous le nom d'Avicel CL-611 par la société FMC Biopolymer, les carbomers dits non sensibles aux électrolytes, vendus sous le nom, d'Ultrez 20®, 1382 ou de Carbopol ETD2020® par la société Noveon, ainsi que l'acrylates/C10-30 Alkyl crosspolymer vendu sous le nom de Pemulen TR-1 ou Pemulen TR-2, les polysaccharides avec à titre d'exemples non limitatifs la gomme de xanthane telle que le Xantural180® vendu par la société Kelco, la gomme guar, les chitosans, la cellulose et ses dérivés tel que l'hydroxypropylméthylcellulose en particulier le produit vendu sous le nom de Methocel E4 premium par la société Dow Chemical ou l'hydroxyéthylcellulose, en particulier, le produit vendu sous le nom de Natrosol HHX 250® par la société Aqualon ou encore le produit « microcrystalline cellulose and carboxymethyl cellulose sodium » vendu sous le nom d'Avicel CL-611 par la société FMC Biopolymer,, la famille des aluminium magnésium silicates tel que le Veegum K vendu par la société Vanderbilt ,la famille des carrhaghénanes en particulier réparties sous quatre grandes familles : k, À, β, w tel que les Viscarin ® et Gelcarins ® commercialisés par la société IMCD, la famille des polymères acryliques couplés à des chaînes hydrophobes tel que le PEG-150/decyl/SMDI copolymer vendu sous le nom de Aculyn 44 (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%)), la famille des amidons modifiés tels que l'amidon de pomme de terre modifié vendu sous le nom de Structure Solanace ou bien leurs mélanges et les gélifiants de la famille des polyacrylamides tels que le mélange Sodium acryloyldimethyltaurate copolymer / isohexadecane / polysorbate 80 vendu sous le nom Simulgel 600 PHA par la société Seppic, le mélange polyacrylamide / isoparaffine C13-14 / laureth-7 comme, par exemple, celui vendu sous le nom de Sepigel 305 par la société Seppic,.

Les gélifiants préférés sont issus de la famille des polyacrylamides tel que le Simulgel 600 ou le Sepigel 305 ; des carbomers dits non sensibles aux électrolytes tel que le Carbopol ETD2020 NF; des polysaccharides tel que la gomme de xanthane ; des dérivés cellulose tel que l'hydroxypropylméthylcellulose ou l'hydroxyéthylcellulose ; des aluminium magnésium silicates seuls ou en mélange.

Les gélifiants et/ou agents de suspension et/ou agents gélifiants pH indépendant, tels que décrits ci-dessus peuvent être utilisés aux concentrations préférentielles allant de 0,001 à 15 % et, plus préférentiellement, allant de 0,1 à 5 %.

La composition peut comprendre en outre tout additif usuellement utilisé dans le domaine cosmétique ou pharmaceutique, tel que des antioxydants, des filtres solaires, des conservateurs, des charges, des électrolytes, des humectants et/ou émollients, des colorants, des agents neutralisants de type bases ou acides usuels, minéraux ou organiques (à titre d'exemple, la triethanolamine, la solution de soude à 10%, le tampon acide succinique/succinate de sodium, le tampon acide citrique/citrate de sodium), des parfums, des huiles essentielles, des actifs cosmétiques, des hydratants, des vitamines, des acides gras essentiels, des sphingolipides, des composés auto-bronzants tels que la DHA, des agents apaisants et protecteurs de la peau tels que l'allantoïne, des agents pro pénétrants, ou un mélange de ceux-ci, optionnellement un stabilisant du peroxyde de benzoyl (à titre d'exemple le docusate de sodium, le sodium C14-16 oléfine sulfonate). Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées.

Ces additifs peuvent être présents dans la composition à raison de 0,001 à 20 % en poids par rapport au poids total de la composition.

On peut citer à titre d'exemples de conservateurs le chlorure de benzalkonium, le bronopol, la chlorhexidine, le chlorocrésol et ses dérivés, l'alcool éthylique, l'alcool phénéthylique, le phénoxyéthanol, le sorbate de potassium, la diazolidinylurée, le chlorure de benzalkonium, le phénoxyéthanol, l'alcool benzylique, la diazolidinylurée, les parabens, ou leurs mélanges.

On peut citer comme exemples d'agents humectants et/ou emollients, la glycérine et le sorbitol, Les sucres (à titre d'exemple le glucose, le lactose), les PEG (à titre d'exemple le Lutrol E400), l'urée, les acides aminés (à titre d'exemple la sérine, la citrulline, l'alamine).

En particulier, l'invention concerne également une composition pharmaceutique ou cosmétique pour application topique sur la peau, les phanères ou les muqueuses, sous forme d'une émulsion comprenant, dans un milieu physiologiquement acceptable, les ingrédients (exprimé en pourcentage en poids) choisis parmi:
- de 0.001 et 5%, préférentiellement 0,01% à 0,5% d'un rétinoïde et de préférence d'un dérivé de l'acide naphtoïque ;
- de 0,025 à 10 %, préférentiellement 2 à 10% de peroxyde de benzoyle ;
- de 30% à 95% préférentiellement 50% à 85% d'eau;
- de 0,01% à 15% préférentiellement 0.1 % à 5% d'agents gélifiants et/ ou de suspension et/ou d'agent gélifiants pH indépendants ;
- de 1% à 15% préférentiellement 3% à 11 % d'émulsionnants;
- de 2% à 50% préférentiellement 5% à 30% de phase grasse ;
- de 0% à 1,5% préférentiellement 0,05% à 0.5% d'un ou plusieurs agents chélatants ;
- de 0 % à 10% préférentiellement 2% à 7% d'un ou plusieurs agents mouillants ;
- de 0.1 à 20% préférentiellement 2% à 15% d'un ou plusieurs agents humectants et/ou emollients ;
- de 0% à 3% préférentiellement 0,05% à 1% d'agents conservateurs ;
- de 0 à 3% préférentiellement 0,05% à 2 % d'agents stabilisants ;
- de 0% à 10% préférentiellement 0,1 % à 5 % d'agents neutralisants ;

La présente invention a aussi pour objet la composition telle que décrite précédemment à titre de médicament.

L'invention se rapporte également à l'utilisation de la nouvelle composition telle que décrite précédemment en cosmétique et en dermatologie.

De part l'activité kératolytique, bactéricide et anti-inflammatoire du péroxyde de benzoyle et l'activité marquée des rétinoïdes dans les domaines de la différenciation et de la prolifération cellulaire, les compositions de l'invention conviennent particulièrement bien dans les domaines thérapeutiques suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle, l'hidradenite supurative,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation telles que les folliculites,
4) pour traiter toutes les proliférations dermiques ou épidermiques qu'elles soient bénignes ou malignes, qu'elles soient ou non d'origine virale telles que verrues vulgaires, les verrues planes, le molluscum contagiosum, et l'épidermodysplasie verruciforme, les papillomatoses orales ou florides et les proliférations pouvant être induites par les ultra-violets notamment dans le cas des kératoses actiniques,
5) pour réparer ou lutter contre le vieillissement de la peau, qu'il soit photo-induit ou chronologique, ou pour réduire les pigmentations, ou toutes pathologies associées au vieillissement chronologique ou actinique,
6) pour traiter de manière préventive ou curative les troubles de la cicatrisation, les ulcères cutanés, pour prévenir ou pour réparer les vergetures, ou encore pour favoriser la cicatrisation,
7) pour lutter contre les troubles de la fonction sébacée tels que l'hyperséborrhée de l'acné ou la séborrhée simple,
8) dans le traitement de toute affection d'origine fongique au niveau cutané tel que le tinea pedis et le tinea versicolor,
9) dans le traitement d'affections dermatologiques à composante immunologique,
10) dans le traitement de désordres cutanés dus à une exposition aux rayonnements U.V., et
11) dans le traitement d'affections dermatologiques liées à une inflammation ou une infection des tissus environnants le follicule pileux, notamment dues à une colonisation ou infection microbienne notamment l'impétigo, la dermite seborrhéique, la folliculite, le sycosis barbae ou impliquant tout autre agent bactérien ou fongique.

Les compositions selon l'invention sont particulièrement adaptées au traitement, de manière préventive ou curative, des acnés vulgaires.

Un objet de l'invention se rapporte également à la préparation d'une composition pharmaceutique destinée à la prévention et/ou au traitement des affections dermatologiques liées à des désordres de la différentiation et/ou de la prolifération cellulaire et/ou de la kératinisation, de préférence les acnés vulgaires.

Les compositions selon l'invention trouvent également une application dans le domaine cosmétique, en particulier pour le traitement des peaux à tendance acnéique, pour la repousse des cheveux, l'anti-chute, pour lutter contre l'aspect gras de la peau ou des cheveux, dans la protection contre les aspects néfastes du soleil ou pour prévenir et/ou pour lutter contre le vieillissement photo-induit ou chronologique.

Les compositions selon l'invention trouvent aussi une application dans l'hygiène corporelle et capillaire.

Préférentiellement, lesdites compositions selon l'invention sont administrées par voie topique.

L'invention a également pour objet un procédé de préparation d'une composition telle que décrite précédemment. Un tel procédé est caractérisé en ce qu'il comprend une étape de mélange d'un milieu physiologiquement acceptable avec au moins un dérivé de l'acide naphtoïque et du péroxyde de benzoyle.

L'introduction des autres excipients et additifs éventuels se fera en fonction de la nature chimique des composés et de la forme galénique choisie.

D'une manière générale, la préparation d'une composition selon l'invention se fait à titre d'exemple selon le procédé principal suivant:
a) mélange d'au moins un rétinoïde avec de l'eau, jusqu'à parfaite dispersion, afin d'obtenir la phase active 1;
b) mélange du péroxyde de benzoyle avec de l'eau, jusqu'à parfaite dispersion, afin d'obtenir la phase active 2;
c) mélange d'au moins un composé hydrophile avec de l'eau, afin d'obtenir la phase aqueuse ;
d) mélange d'au moins un émulsionnant avec un composé lipophile afin d'obtenir la phase grasse ;
e) introduction de la phase grasse obtenue en d) dans la phase aqueuse obtenue en c) afin d'obtenir une émulsion ;
f) introduction des phases actives 1 et 2 respectivement obtenues en a) et b) dans l'émulsion obtenue en e).
g) si nécessaire, un agent de neutralisation du gélifiant est introduit dans l'émulsion obtenu en e);
h) si nécessaire, les additifs thermosensibles sont ajoutés ;
i) si nécessaire un complément d'eau est ajouté.

D'une manière générale, la préparation d'une composition selon l'invention se fait à titre d'exemple selon le procédé alternatif suivant:
a') Les étapes a) et b) sont regroupées de manière à obtenir l'étape a') qui correspond au mélange d'au moins un rétinoïde, de benzoyle peroxide et d'au moins un agent mouillant avec de l'eau, jusqu'à parfaite dispersion, afin d'obtenir une seule phase active. Les étapes c), d),e), f), g), h), i), j) du procédé principal restent inchangées.

De manière préférée, la préparation d'une composition selon l'invention se fait à titre d'exemple selon le procédé principal suivant:
a) Le rétinoïde, de préférence le dérivé d'acide naphtoique est mélangé avec au moins un agent mouillant dans de l'eau, jusqu'à parfaite dispersion de l'actif, afin d'obtenir la phase active 1;
b) Le péroxyde de benzoyle est mélangé avec au moins un agent mouillant, dans de l'eau, jusqu'à parfaite dispersion, afin d'obtenir la phase active 2;
c) On solubilise dans l'eau sous agitation , si nécessaire à chaud, un ou plusieurs agents gélifiants et/ou agent de suspension,et/ou agent gélifiant gélifiant pH indépendant et optionnellement un ou plusieurs agents chélatants, un ou plusieurs conservateurs et les additifs hydrophiles non thermosensibles.,et un ou plusieurs agents humectants et/ou émollients on maintient l'agitation et éventuellement le chauffage jusqu'à homogénéité pour obtenir la phase aqueuse ;
d) On mélange à chaud au moins un émulsionnant, avec des huiles et/ou des corps gras solides, optionnellement avec des conservateurs et les additifs lipophiles non thermosensibles jusqu'à homogénéité afin d'obtenir la phase grasse ;
e) Ladite phase grasse obtenue en d) est introduite dans la phase aqueuse obtenue en c) afin d'obtenir une émulsion ;
f) lesdites phases actives 1 et 2 respectivement obtenues en a) et b) sont introduites dans l'émulsion obtenue en e) afin d'obtenir l'émulsion active ;
g) Si nécessaire, un agent de neutralisation du gélifiant est introduit dans l'émulsion obtenue en f)) afin d'obtenir le pH désiré,
h) Si nécessaire, les additifs thermosensibles sont ajoutés ;
i) Optionnellement le copolymère d'acrylamide de sodium et d'acrylamino-2-méthylpropane sulphonate en dispersion à 40% dans l'isohéxadécane et le polysorbate 80 est ajouté ;
j) Si nécessaire un complément d'eau est ajouté.

De manière préférée, la préparation d'une composition selon l'invention se fait à titre d'exemple selon le procédé alternatif suivant:
a') Les étapes a) et b) sont regroupées de mamière à obtenir l'étape a') qui correspond au mélange d'au moins un rétinoïde, de benzoyle peroxide et d'au moins un agent mouillant avec de l'eau, jusqu'à parfaite dispersion, afin d'obtenir une seule phase active. Les étapes c), d),e), f), g), h), i), j) du procédé principal restent inchangées.

Plus précisément, le procédé principal de préparation de la composition selon l'invention comprend à titre d'exemple les étapes suivantes:

### Etape a: Préparation de la phase active 1:

Dans un bêcher on mélange, le principe actif 1- le rétinoïde (de préférence l'adapalène), une partie de l'eau purifiée, les agents mouillants (type Synperonic PE/L62, Synperonic PE/L44, propylène glycol), on laisse sous agitation jusqu'à parfaite dispersion.;

### Etape b: Préparation de la phase active 2:

Dans un bécher, on introduit sous agitation, l'eau purifiée, le principe actif (Peroxyde de benzoyl), les agents mouillants (type Symperonic PE/L62, Symperonic PE/L44, propylène glycol), on laisse sous agitation jusqu'à parfaire dispersion.

### Etape c: Préparation de la phase aqueuse:

Dans un bécher, on introduit sous agitation, si nécessaire à chaud, de l'eau purifiée et le ou les gélifiants et/ou gélifiants pH indépendants (à l'exception du polyacrylamide) et optionnellement le ou les agents chélatants (type EDTA), le ou les humectants et/ou émollients (type glycérine), le ou les conservateurs (type méthyl paraben), les émulsionnants, le ou les agents de suspension (type Avicel), le ou les agents stabilisants (type docusate de sodium).

### Etape d : Préparation de la phase grasse :

Dans un bêcher,le ou les émulsionnants lipophiles (type Glucate SS, Glucamate SSE 20, Brij 721, Téfose 1500, Eumulgin B2 PH, olépal isostéarique), les composés huileux (type Cetiol SN, Crodamol DA, Speziol C18, Miglyol 812, Cosbiol), les éventuels additifs lipophiles non thermosensibles et éventuellement les conservateurs (type phénoxyéthanol, propyl parabène) sont mélangés.

### Etape e : Emulsification :

A chaud sous agitation, la phase grasse est introduite dans la phase aqueuse afin de réaliser l'émulsification. Le chauffage est maintenu quelques minutes, puis le produit est laissé à refroidir doucement. L'agitation est réglée en fonction de la viscosité. A partir de 50°C on peut introduire le silicone volatile si ce dernier est présent dans la composition.

### Etape f : Incorporation des phases actives 1 et 2 :

A une température inférieure à 40°C, on introduit sous agitation les phases actives 1 et 2 l'une après l'autre. Maintenir l'agitation jusqu'à parfaite homogénéité.

### Etape g (optionnelle): Neutralisation :

A une température inférieure à 40°C, l'agent de neutralisation du gélifiant (type triéthanoloamine ou solution d'hydroxyde de sodium à 10%) ou le tampon pH est introduit si nécessaire, jusqu' au pH désiré. Le produit prend alors une consistance plus épaisse.

A la fin de la fabrication, le pH est à nouveau vérifié. Si nécessaire, l'ajustement à 100% en eau est effectué. Le produit est homogénéisé une dernière fois afin de s'assurer de la bonne dispersion des principes actifs Adapalène et péroxyde de benzoyle (observation microscopique révélant une dispersion homogène et sans agrégats),.

### Etape h (optionnelle): Ajout des autres additifs :

A une température inférieure à 40°C, on introduit sous agitation les éventuels additifs. Maintenir l'agitation jusqu'à parfaite homogénéité.

### Etape i (optionnelle): Ajout du polyacrylamide:

A une température inférieure à 40°C, on introduit sous agitation le polyacrylamide. Maintenir l'agitation jusqu'à parfaite homogénéité ;

### Etape j : Correction de la perte en eau :

On calcule la perte en eau lors de la réalisation du produit et on rajoute sous agitation l'eau perdue, on maintien l'agitation jusqu'à parfaite homogénéité.

Plus précisément, le procédé alternatif de préparation de la composition selon l'invention comprend à titre d'exemple les étapes suivantes:
a') Les étapes a), et b), sont regroupées de mamière à obtenir l'étape a'), qui correspond au mélange d'au moins un rétinoïde, de benzoyle peroxide et d'au moins un agent mouillant avec de l'eau, jusqu'à parfaite dispersion, afin d'obtenir une seule phase active. Les étapes c), d),e), f), g), h), i), j) du procédé principal restent inchangées.

La présente invention va maintenant être illustrée au moyen des exemples suivants et des données de stabilité physique et chimique présentées ci-dessous.

La stabilité physique des formulations est mesurée par une observation macroscopique et microscopique de la formulation à température ambiante et 40°C à T1 mois, T2 mois et optionnellement à T+15jours.

A TA, l'observation macroscopique permet de garantir l'intégrité physique des produits.

L'observation microscopique permet d'évaluer la qualité de la dispersion des deux actifs. L'adapalène est observé en lumière fluorescente alors que le peroxyde de benzoyl est observé en lumière polarisée.

On complète la caractérisation du produit fini par une mesure du seuil d'écoulement et de viscosité.

Pour la mesure du seuil d'un rhéomètre HAAKE de type VT550 avec un mobile de mesure SVDIN.

Les rhéogrammes sont réalisés à 25°C et à la vitesse de cisaillement de 4 s⁻¹ , 20s-1, 100s-1 (γ), et en mesurant la contrainte de cisaillement. Par seuil d'écoulement (τ0 exprimé en Pascal) on entend la force nécessaire (contrainte de cisaillement minimum) pour vaincre les forces de cohésion de type Van der Waals et provoquer l'écoulement.

Le seuil d'écoulement est assimilé à la valeur trouvée à la vitesse de cisaillement de 4s-1.

Pour les mesures de viscosité, On utilise des viscosimètres de type Brookfield RVDVII+ et LDVDII+.

Les gammes de viscosité mesurables avec les 2 types de Brookfield sont les suivantes : Viscosimètre RVDVII+ : 100 cP - 40 McP

Viscosimètre LVDVII+ / 15 cP - 6 McP

On considère que l'on a à l'instant initial T0 :
Une crème si la viscosité est supérieure à 30 000 cP
Une lotion si la viscosité est inférieure à 30 000 cP (Lucinda Bushe, ACPS October 22, 2003 Pharmaceutical nomenclature-Issue and challenges).
La stabilité chimique est assurée par un dosage HPLC des actifs.
Le résultat est exprimé en g/g d'apalène et de peroxyde de benzoyle, et en % par rapport au titre attendu.

Les exemples de formulations ci-dessous permettent d'illustrer les compositions selon l'invention, sans toutefois en limiter la portée. Des exemples de procédés de préparation des compositions selon l'invention, mentionnés à titre non limitatif.

### Exemple 1 : Formulation de type crème contenant de l'adapalène à 0,1% et du péroxyde de benzoyle à 2.5%

La formule est préparée selon le mode opératoire décrit plus haut

| **Constituants** | **Teneur (% m/m)** |
|---|---|
| Péroxyde de benzoyle | 2,50 |
| Adapalène | 0,10 |
| Propylène glycol | 4,00 |
| Synperonic PE/L44 | 0,20 |
| Docusate de sodium | 0,05 |
| Propylène glycol | 2,00 |
| EDTA | 0,10 |
| Carbopol Ultrez 20 | 0,40 |
| Glycérine | 3,00 |
| Glucamate SSE 20 | 3,50 |
| Glucate SS | 3,50 |
| Cosbiol | 6,00 |
| ST-Cyclomethicone 5 NF | 13,00 |
| Eau purifiée | qsp 100 |
| Triéthanolamine | qsp pH 5,5±0,5 |

### Données de stabilités :

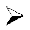 Stabilité physique :

| **Caractérisations à TO** | | |
|---|---|---|
| Aspect macroscopique | | Crème blanche |
| Aspect microscopique | | Dispersion des actifs sans agrégats > 100 µm |
| pH | | 6.07 |
| Données de viscosité | Haake (4s-1/20s-1/100s-1) | 233/312/418 |
| | Brookfield RVDVII+ (S29 ; 5rpm) | 184990 cP |

| | | T+15 Jours | T+1 mois | T+2mois | T+3mois |
|---|---|---|---|---|---|
| Aspect macroscopique | TA | Identique à T0 | Identique à T0 | Identique à T0 | Identique à T0 |
| | 40°C | Identique à T0 | Identique à T0 | Identique à T0 | Identique à T0 |
| Aspect microscopique | TA | Identique à T0 | Identique à T0 | Identique à T0 | Identique à T0 |
| | 40°C | Identique à T0 | Identique à T0 | Identique à T0 | Identique à T0 |
| pH | TA | 5.98 | 5.96 | 5.87 | 5.84 |
| | 40°C | 5.56 | 5.37 | 4.81 | 4.55 |
| Rhéologie Haake 4s-1/20s-1/100s-1 | | N.R | 223/310/379 | 245/328/385 | N.R |
| Brookfield RVDVII+ (S29 ; 5rpm) | | NR | 196042 cP | 189880 cP | 187610cP |

### STABILITE CHIMIQUE:

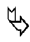 **ADAPALENE**

| Temps → | | T0 | T1M | T2M |
|---|---|---|---|---|
| Conditions de stabilité↓ | | | | |
| TA | g/g | 0.10 | 0.10 | 0.10 |
| | % du titre attendu | 100% | 100% | 100% |
| 40°C | g/g | N.A | 0.10 | 0.10 |
| | % du titre attendu | | 100% | 100% |

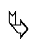 **BENZOYL PEROXIDE**

| Temps → | | T0 | T1M | T2M |
|---|---|---|---|---|
| Conditions de stabilité↓ | | | | |
| TA | g/g | 2.60 | 2.6 | 2.6 |
| | % du titre attendu | 104% | 104% | 104% |
| 40°C | g/g | N.A | 2.4 | 2.3 |
| | % du titre attendu | | 96% | 92% |

### Exemple 2 : Formulation de type crème contenant de l'adapalène à 0,3% et et du péroxyde de benzoyle à 5%

La formule est préparée selon le mode opératoire décrit plus haut

| **Constituants** | **Teneur (% m/m)** |
|---|---|
| Péroxyde de benzoyle | 5,00 |
| Adapalène | 0,30 |
| Dipropylène glycol | 5,00 |
| Synperonic PE/L44 | 0,20 |
| Glycérine | 7,00 |
| Xantural 180 | 0,40 |
| Eumulgin B2 PH | 3,00 |
| Arlacel 165FL | 3,00 |
| Speziol C18 Pharma | 2,00 |
| Mygliol 812 N | 7,00 |
| ST-Cyclomethine 5 NF | 6,00 |
| Simulgel 600 PHA | 2,50 |
| Eau purifiée | qsp 100 |
| Hydroxyde de sodium | qsp pH 5,5±0,5 |

### Données de stabilités :

➢ Stabilité physique :

| **Caractérisations à TO** | | |
|---|---|---|
| Aspect macroscopique | | Crème blanche |
| Aspect microscopique | | Dispersion des actifs sans agrégats > 100 µm |
| pH | | 6.18 |
| Données de viscosité | Haake (4s-1/20s-1/100s1) | 135/189/311 |
| | Brookfield RVDVII+ (S29 ; 5rpm) | 99810 cP |

| | | T+15 Jours | T+1 mois | T+2mois | T+3mois |
|---|---|---|---|---|---|
| Aspect macroscopique | TA | Identique à T0 | Identique à T0 | Identique à T0 | Identique à T0 |
| | 40°C | Identique à T0 | Identique à T0 | Identique à T0 | Identique à T0 |
| Aspect microscopique | TA | Identique à T0 | Identique à T0 | Identique à T0 | Identique à T0 |
| | 40°C | Identique à T0 | Identique à T0 | Identique à T0 | Identique à T0 |
| pH | TA | 5.78 | 5.39 | 5.35 | 5.14 |
| | 40°C | 4.36 | 4.12 | 3.82 | 3.74 |
| Rhéologie Haake 4s-1/ 20s-1/100s-1 | | N.R | N.R | 117/190/287 | N.R |
| Brookfield RVDVII+ (S29 ; 5rpm) | | NR | NR | 90310 cP | 94580cP |

### STABILITE CHIMIQUE:

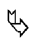 **ADAPALENE**

| Temps → | | T0 | T1M |
|---|---|---|---|
| Conditions de stabilité↓ | | | |
| TA | g/g | 0.29 | 0.29 |
| | % du titre attendu | 96.66% | 96.66% |
| 40°C | g/g | N.A | 0.29 |
| | % du titre attendu | | 96.66% |

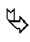 **BENZOYL PEROXIDE**

| Temps → | | T0 | T1M |
|---|---|---|---|
| Conditions de stabilité↓ | | | |
| TA | g/g | 5.10 | 5.10 |
| | % du titre attendu | 102% | 102% |
| 40°C | g/g | N.A | 4.70 |
| | % du titre attendu | | 94% |

### Exemple 3 Formulation de type crème contenant de l'adapalène à 0,1% et et du péroxyde de benzoyle à 5%

La formule est préparée selon le mode opératoire décrit plus haut

| **Constituants** | **Teneur (% m/m)** |
|---|---|
| Péroxyde de benzoyle | 5,00 |
| Adapalène | 0,10 |
| Propylène glycol | 6,00 |
| Synperonic PE/L44 | 0,20 |
| EDTA | 0,10 |
| Glycérine | 7,0 |
| Veegum K | 0,20 |
| Natrosol HHX | 0,20 |
| Eumulgin B2 PH | 3,0 |
| Speziol C18 pharma | 2,0 |
| Miglyol 812 N | 7,0 |
| Arlacel 165FL | 3,0 |
| ST-Cyclomethicone 5 NF | 6,0 |
| Simulgel 600 | 2,0 |
| Eau purifiée | qsp 100 |
| Triethanolamine | qsp pH 5,5±0,5 |

### Données de stabilités :

➢ Stabilité physique :

| **Caractérisations à T0** | | |
|---|---|---|
| Aspect macroscopique | | Crème blanche |
| Aspect microscopique | | Dispersion des actifs sans agrégats > 100 µm |
| pH | | 6.68 |
| Données de viscosité | Haake (4s-1/20s-1/100s-1) | 82/112/178 |

| | | T+15 Jours | T+1 mois | T+2mois | T+3mois |
|---|---|---|---|---|---|
| Aspect macroscopique | TA | Identique à T0 | Identique à T0 | Identique à T0 | Identique à T0 |
| | 40°C | Identique à T0 | Identique à T0 | Identique à T0 | Identique à T0 |
| Aspect microscopique | TA | Identique à T0 | Identique à T0 | Identique à T0 | Identique à T0 |
| | 40°C | Identique à T0 | Identique à T0 | Identique à T0 | Identique à T0 |
| pH | TA | 5.80 | 5.85 | 5.59 | 5.47 |
| | 40°C | NR | 4.41 | 4.13 | 3.82 |
| Rhéologie Haake 4s-1/ 20s-1/100s-1 | | N.R | N.R | 69/108/168 | 61/95/168 |

### Exemple 4 : Formulation de type crème contenant de l'adapalène à 0,3% et et du péroxyde de benzoyle à 5%

La formule est préparée selon le mode opératoire décrit plus haut

| **Constituants** | **Teneur (% m/m)** |
|---|---|
| Péroxyde de benzoyle | 5,00 |
| Adapalène | 0,30 |
| Dipropylène glycol | 5,00 |
| Synperonic PE/L44 | 0,20 |
| Glycérine | 7,00 |
| Xantural 180 | 0,40 |
| Eumulgin B2 PH | 3,00 |
| Arlacel 165FL | 3,00 |
| Speziol C18 Pharma | 2,00 |
| Mygliol 812 N | 7,00 |
| ST-Cyclomethicone 5-NF | 6,00 |
| Simulgel 600 PHA | 2,50 |
| Eau purifiée | qsp 100 |
| Tampon acide succinique+ | |
| succinate de sodium | qsp pH 5,5±0,5 |

### Données de stabilités :

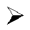 Stabilité physique :

| **Caractérisations à TO** | | |
|---|---|---|
| Aspect macroscopique | | Crème blanche |
| Aspect microscopique | | Dispersion des actifs sans agrégats > 100 µm |
| pH | | 5.06 |
| Données de viscosité | Haake (4s-1/20s-1/100s-1) | 103/154/253 |

| | | T+15 Jours | T+1 mois | T+2mois | T+3mois |
|---|---|---|---|---|---|
| Aspect macroscopique | TA | Identique à T0 | Identique à T0 | Identique à T0 | Identique à T0 |
| | 40°C | Identique à T0 | Identique à T0 | Identique à T0 | Identique à T0 |
| Aspect microscopique | TA | Identique à T0 | Identique à T0 | Identique à T0 | Identique à T0 |
| | 40°C | Identique à T0 | Identique à T0 | Identique à T0 | Identique à T0 |
| pH | TA | 4.86 | 4.82 | 4.66 | 4.59 |
| | 40°C | NR | 3.80 | 3.62 | 3.45 |
| Rhéologie Haake 4s-1/ 20s-1/100s-1 | | N.R | N.R | 72/123/215 | 97/156/245 |

### Exemple 5 : Formulation de type lotion contenant de de l'adapalène à 0,3% et du péroxyde de benzoyle à 1%

La formule est préparée selon le mode opératoire décrit ci-dessus :

| **Constituants** | **Teneur (% m/m)** |
|---|---|
| Péroxyde de benzoyle | 1,00 |
| Adapalène | 0,30 |
| Avicel CL-611 | 1.50 |
| Dipropylene Glycol | 3.00 |
| Synperonic PE/L44 | 0,20 |
| Méthyl paraben | 0,15 |
| Brij 721 | 3,00 |
| Arlacel 165FL | 3,00 |
| Propyl paraben | 0,05 |
| Perhydrosqualene | 5,00 |
| Cetiol SN PH | 5,00 |
| Simulgel 600 PHA | 1.50 |
| Eau purifiée | qsp 100 |

Triethanolamine qsp pH 5,5±0,5

### Données de stabilités :

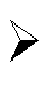 Stabilité physique :

| **Caractérisations à T0** | | |
|---|---|---|
| Aspect macroscopique | | Lotion blanche |
| Aspect microscopique | | Dispersion des actifs sans agrégats > 100 µm |
| pH | | 5.534 |
| Données de viscosité | Haake (4s-1/20s-1/100s-1) | 23/40/88 |
| | Brookfield LVDVII+ (5rpm; S63) | 21283cP |

| | | T+1 mois | T+2mois |
|---|---|---|---|
| Aspect macroscopique | TA | Identique à T0 | Identique à T0 |
| | 40°C | Identique à T0 | Identique à T0 |
| Aspect microscopique | TA | Identique à T0 | Identique à T0 |
| | 40°C | Identique à T0 | Identique à T0 |
| pH | TA | 5.00 | 4.75 |
| | 40°C | 4.09 | 3.87 |
| Rhéologie Haake 4s-1/ 20s-1/100s-1 | | 18/35/69 | 17/27/63 |
| Viscosité Brookfield LVDVII+ ; (5rpm; S63) | | 15861cP | 14637cP |

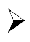 Stabilité Chimique :

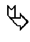 **Adapalène**

| Temps → | | T0 | T+1 mois |
|---|---|---|---|
| Conditions de stabilité↓ | | | |
| TA | g/g | 0.29 | 0.29 |
| | % titre attendu | 97 | 97 |
| 40°C | g/g | NA | 0.28 |
| | % titre attendu | NA | 93 |

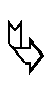 **Benzoyl peroxide**

| Temps → | | T0 | T+1 mois |
|---|---|---|---|
| Conditions de stabilité↓ | | | |
| TA | g/g | 1.2 | 1.2 |
| | % titre attendu | 120 | 120 |
| 40°C | g/g | NA | 1.0 |
| | % titre attendu | NA | 100 |

## Revendications

1. Composition comprenant, dans un milieu physiologiquement acceptable, au moins un rétinoïde et du péroxyde de benzoyle dispersé, **caractérisée en ce qu'**elle se présente sous forme d'émulsion.

2. Composition selon la revendication 1, **caractérisée en ce qu'**elle comprend, outre le rétinoïde et le péroxyde de benzoyle :
- au moins une phase hydrophile ;
- au moins une phase grasse ;
- au moins un émulsionnant.

3. Composition selon la revendication 2, **caractérisée en ce que** la phase hydrophile est une phase aqueuse.

4. Composition selon l'une quelconque des revendications précédentes, **caractérisée en ce qu'**elle est stable physiquement et chimiquement.

5. Composition selon l'une des revendications 3 ou 4, **caractérisée en ce que** l'émulsionnant est choisi parmi le glycéryl stéarate et PEG-100 stéarate, le PEG-6 stéarate et PEG-12 stéarate, les esters de sucrose, le cétéareth 20, et le polyoxyéthylène (21) stéaryl éther.

6. Composition selon l'une des revendications 1 à 5, **caractérisée en ce qu'**elle comprend entre 0,0001 et 20 % de rétinoïde.

7. Composition selon l'une des revendications 1 à 6, **caractérisée en ce que** le rétinoïde est l'adapalène.

8. Composition selon l'une des revendications 1 à 7, **caractérisée en ce qu'**elle comprend entre 0,0001 et 20 % de péroxyde de benzoyle.

9. Composition selon l'une des revendications 1 à 8, **caractérisée en ce que** le péroxyde de benzoyle est sous forme encapsulée ou libre.

10. Composition selon l'une des revendications 2 à 9, **caractérisée en ce qu'**elle comprend entre 0,001 et 20 % d'émulsionnants en poids par rapport au poids total de la composition.

11. Composition selon l'une quelconque des revendications précédentes, **caractérisée en ce qu'**elle comprend un agent mouillant.

12. Composition selon la revendication 11, **caractérisée en ce qu'**elle comprend entre 0,001 et 20 % d'agent mouillant.

13. Composition selon la revendication 11 ou 12, **caractérisée en ce que** l'agent mouillant est le propylène glycol ou le Synperonic PE/L44.

14. Composition selon l'une quelconque des revendications précédentes, **caractérisée en ce qu'**elle comprend :
- de 0.001 et 5%, préférentiellement 0,01% à 0,5% d'un rétinoïde et de préférence d'un dérivé de l'acide naphtoïque ;
- de 0,025 à 10 %, préférentiellement 2 à 10% de péroxyde de benzoyle ;
- de 30% à 95% préférentiellement 50% à 85% d'eau;
- de 0,01% à 15% préférentiellement 0.1 % à 5% d'agents gélifiants et/ ou de suspension et/ou d'agent gélifiants pH indépendants ;
- de 1% à 15% préférentiellement 3% à 11 % d'émulsionnants;
- de 2% à 50% préférentiellement 5% à 30% de phase grasse ;
- de 0% à 1,5% préférentiellement 0,05% à 0.5% d'un ou plusieurs agents chélatants ;
- de 0 % à 10% préférentiellement 2% à 7% d'un ou plusieurs agents mouillants ;
- de 0.1 à 20% préférentiellement 2% à 15% d'un ou plusieurs agents humectants et/ou emollients ;
- de 0% à 3% préférentiellement 0,05% à 1% d'agents conservateurs ;
- de 0 à 3% préférentiellement 0,05% à 2 % d'agents stabilisants ;
- de 0% à 10% préférentiellement 0,1 % à 5 % d'agents neutralisants ;

15. Composition selon l'une quelconque des revendications de 1 à 14 à titre de médicament.

16. Procédé de préparation de la composition selon l'une quelconque des revendications 1 à 14, **caractérisé en ce qu'**il comprend les étapes suivantes:
a) mélange d'au moins un rétinoïde avec de l'eau, jusqu'à parfaite dispersion, afin d'obtenir la phase active 1;
b) mélange du péroxyde de benzoyle avec de l'eau, jusqu'à parfaite dispersion, afin d'obtenir la phase active 2;
c) mélange d'au moins un composé hydrophile avec de l'eau, afin d'obtenir la phase aqueuse ;
d) mélange d'au moins un émulsionnant avec un composé lipophile afin d'obtenir la phase grasse ;
e) introduction de la phase grasse obtenue en d) dans la phase aqueuse obtenue en c) afin d'obtenir une émulsion ;
f) introduction des phases actives 1 et 2 respectivement obtenues en a) et b) dans l'émulsion obtenue en e).
g) si nécessaire, un agent de neutralisation du gélifiant est introduit dans l'émulsion obtenu en e);
h) si nécessaire, les additifs thermosensibles sont ajoutés ;
i) si nécessaire un complément d'eau est ajouté.

17. Procédé de préparation de la composition selon la revendication 16, **caractérisé en ce qu'**il comprend les étapes suivantes:
a) Le rétinoïde, de préférence le dérivé d'acide naphtoique est mélangé avec au moins un agent mouillant dans de l'eau, jusqu'à parfaite dispersion de l'actif, afin d'obtenir la phase active 1;
b) Le péroxyde de benzoyle est mélangé avec au moins un agent mouillant, dans de l'eau, jusqu'à parfaite dispersion, afin d'obtenir la phase active 2;
c) On solubilise dans l'eau sous agitation , si nécessaire à chaud, un ou plusieurs agents gélifiants et/ou agent de suspension,et/ou agent gélifiant gélifiant pH indépendant et optionnellement un ou plusieurs agents chélatants, un ou plusieurs conservateurs et les additifs hydrophiles non thermosensibles.,et un ou plusieurs agents humectants et/ou émollients on maintient l'agitation et éventuellement le chauffage jusqu'à homogénéité pour obtenir la phase aqueuse ;
d) On mélange à chaud au moins des émulsionnants, des huiles et/ou des corps gras solides avec optionnellement des conservateurs et autres additifs lipophiles non thermosensibles jusqu'à homogénéïté afin d'obtenir la phase grasse ;
e) Ladite phase grasse obtenue en d) est introduite dans la phase aqueuse obtenue en c) afin d'obtenir une émulsion ;
f) les dites phases actives 1 et 2 respectivement obtenues en a) et b) sont introduites dans l'émulsion obtenue en e) afin d'obtenir l'émulsion active ;
g) Si nécessaire, un agent de neutralisation du gélifiant est introduit dans l'émulsion obtenue en f) afin d'obtenir le pH désiré ;
h) Si nécessaire, les additifs thermosensibles sont ajoutés.

18. Procédé de préparation de la composition selon la revendication 17, **caractérisé en ce qu'**il comprend une étape d'ajout, après l'étape h), du copolymère d'acrylamide de sodium et d'acrylamino-2-méthylpropane sulphonate en dispersion à 40% dans l'isohéxadécane et le polysorbate 80.

19. Utilisation d'une composition selon l'une quelconque des revendications de 1 à 14 pour la fabrication d'une préparation pharmaceutique destinée à prévenir ou à traiter les affections dermatologiques liées à des désordres de la différentiation et/ou la prolifération cellulaire et/ou de la kératinisation.

20. Utilisation d'une composition selon l'une quelconque des revendications de 1 à 14 pour fabriquer une préparation pharmaceutique destinée à prévenir ou à traiter les acnés vulgaires.

21. Utilisation cosmétique d'une composition selon l'une quelconque des revendications 1 à 13 pour le traitement des peaux à tendance acnéique, pour faire repousser les cheveux ou éviter leur chute, pour lutter contre l'aspect gras de la peau ou des cheveux, dans la protection contre les aspects néfastes du soleil ou pour prévenir et/ou lutter contre le vieillissement photo-induit ou chronologique.
